Europäisches Patentamt

European Patent Office

Office européen des brevets

(19)

(11) Publication number: **0 091 708**
**B1**

(12) # EUROPEAN PATENT SPECIFICATION

(45) Date of publication of patent specification: **29.07.87**

(51) Int. Cl.⁴: **C 07 C 19/05, C 07 C 17/42**

(21) Application number: **83200456.8**

(22) Date of filing: **31.03.83**

(54) Stabilized 1,1,1-trichlorethane.

(30) Priority: **09.04.82 AT 1420/82**

(43) Date of publication of application:
**19.10.83 Bulletin 83/42**

(45) Publication of the grant of the patent:
**29.07.87 Bulletin 87/31**

(84) Designated Contracting States:
**AT CH DE FR GB IT LI NL SE**

(56) References cited:
**GB-A-2 024 243**
**US-A-3 629 128**
**US-A-4 018 837**

**CHEMICAL ABSTRACTS, vol. 86, 1977, page 499, no. 120773b, Columbus Ohio USA;**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Gregor, Reinhard**
**Rennweg 12**
**A-1030 Vienna (AT)**
Inventor: **Ruzha, Wolfgang**
**Rennweg 12**
**A-1030 Vienna (AT)**
Inventor: **Hönig, Werner**
**Rennweg 12**
**A-1030 Vienna (AT)**

(74) Representative: **Aalbers, Onno et al**
**P.O. Box 302**
**NL-2501 CH The Hague (NL)**

EP 0 091 708 B1

Courier Press, Leamington Spa, England.

## Description

The invention relates to stabilized 1,1,1-trichloroethane and to a stabilizer composition suitable to stabilize 1,1,1-trichloroethane.

1,1,1-Trichloroethane is a technical solvent widely used on account of its low toxicity and good ecological properties. One of the most important fields of application of 1,1,1-trichloroethane is the cleaning and/or degreasing of metal objects.

It is known that chlorinated hydrocarbons such as perchloroethylene, trichloroethylene, 1,1,1-trichloroethane and other aliphatic halogen hydrocarbons readily decompose. 1,1,1-Trichloroethane, however, decomposes particularly readily in the presence of metals, in particular of light metals. Light metals, such as aluminium and aluminium alloys, catalyse said decomposition—particularly in the presence of heat—to a high degree, with hydrogen chloride and other decomposition products being formed. Decomposition takes place autocatalytically in the manner of a Friedel-Crafts reaction and can sometimes proceed violently. The corrosion, accompanying said decomposition, of the metal objects to be cleaned or on the degreasing equipment is extremely undesirable. For industrial use, therefore, 1,1,1-trichloroethane is only employed with added stabilizers.

For the cleaning of metals, stabilized 1,1,1-trichloroethane is used to remove lubricating oils, greases or greasy compounds at either normal temperature or boiling point.

Reliable stabilization, however is particularly difficult if the 1,1,1-trichloroethane is used at boiling temperatures, as is the case with vapour degreasing equipment.

To achieve a permanent stabilizer effect during both cold and hot application of 1,1,1-trichloroethane, suitable selection of the stabilizer constituents with a view to their effectiveness in both the liquid phase and the vapour phase is essential.

For the stabilizing of 1,1,1-trichloroethane, the most varied chemical compounds and mixtures have in the past been proposed, such as nitroalkanes, amines, nitriles, alcohols, ketones, aldehydes, olefins, glycols, alkoxyalkanes and ethers.

The known stabilizer mixtures must, however, be added in relatively large quantities to achieve an adequate effect. Even then the known stabilizer mixtures do not always satisfy the strict requirements imposed on the stabilizer effect in various test methods.

It was therefore the aim of the invention to find a 1,1,1-trichloroethane stabilizer composition of which the individual constituents have a synergistic effect on stabilization and exhibit sufficient effectiveness during hot and cold degreasing. In addition, a 1,1,1-trichloroethane stabilized according to the invention was intended to satisfy the extremely strict requirements of the BAM test method in all respects.

A particularly stringent test method has been developed by the BAM (Bundesanstalt für Materialprüfung, Berlin) in order to test the long-term stabilizer effect, particularly under the adverse conditions of hot degreasing in the presence of aluminium. This test comprises three different test procedures:

100 ml of stabilized 1,1,1-trichloroethane and 100 ml toluene are both refluxed for 18 h at a bath temperature of 120°C with the following reagents added:

BAM 1: 18g aluminium shavings, 0.7 g AlCl$_3$
BAM 2: as BAM 1 plus addition of 1 g zinc stearate
BAM 3: as BAM 1 plus addition of 10 ml oleic acid.

In a separate test the stabilized 1,1,1-trichloroethane is separated into three fractions of equal magnitude by simple distillation in line with the experimental conditions for fractional analysis according to DIN 51 751. Each of the three fractions is separately examined by the method described under BAM 1.

The test, which constitutes a destructive testing method, is considered to have been passed if no violent exothermic reactions arise during any of the individual tests described.

It has now been found that 1,1,1-trichloroethane can be effectively stabilized by a stabilizer mixture on the basis of tert-butanol, 1,4-dioxane, nitroalkane with 1 to 3 carbon atoms, butylene oxide and/or cyclohexene oxide and aliphatic and/or heterocyclic amines.

The invention relates to stabilized 1,1,1-trichloroethane characterized in that it comprises a combination of

3 —5 % wt. tert-butanol,
1.5 —2.5 % wt. 1,4-dioxane,
0.3 —0.8 % wt. nitroalkane with 1 to 3 carbon atoms,
0.3 —0.8 % wt. butylene oxide and/or cyclohexene oxide and
0.01—0.05% wt. aliphatic and/or heterocyclic amine as stabilizers.

Of the nitroalkanes with 1 to 3 carbon atoms, nitromethane is particularly preferred in the stabilizer combination according to the invention. The amine constituents can, for example, consist of diisopropylamine, triethylamine and/or N-methyl morpholine, with diisopropylamine being preferred.

According to a particularly advantageous embodiment of the present invention, 1,1,1-trichloroethane is stabilized with 3.7% wt. tert.-butanol, 1.8% wt. 1,4-dioxane, 0.5% wt. 1,2-butylene oxide, 0.6% wt. nitromethane and 0.02% wt. diisopropylamine, each in relation to the total composition.

Although the individual constituents of the stabilizer combination according to the invention have

2

already been proposed in a number of publications, the advantageous effect of the novel synergistic combination has not hitherto been recognized.

The constituents contained in the stabilizer mixture according to the invention are not effective when used individually; neither combinations of 1,4-dioxane, nitromethane and butylene oxide or of tert-butanol, 1,4-dioxane and butylene oxide pass the stringent BAM test.

Furthermore, from the German Published Application 29 26 000 is known the stabilizing of 1,1,1-trichloroethane by a combination of the following additives: 0.1 to 5% wt. tert-butanol, 0.1 to 5% wt. trioxane, 0.1 to 5% wt. epoxide, 0.1 to 3% wt. nitroalkane and 0.001 to 1% wt. amine as well as at least one special antioxidant if necessary. In example 7 of said Published Application, according to an embodiment designated as advantageous the sole cyclic ether 1,3,5-trioxane is added as one of the five obligatory constituents.

There are, however, a number of reasons for not using trioxane as cyclic ether constituent.

Trioxane is a formaldehyde derivative and sublimes when heated. At boiling temperature of, 1,1,1-trichloroethane particularly in the presence of acids, trioxane depolymerizes to formaldehyde and/or paraformaldehyde. Because of the volatility of the decomposition products, no long-term stabilizer effect can be guaranteed if 1,3,5-trioxane is used as the sole cyclic ether.

In comparative experiments this unsatisfactory behaviour has been clearly documented. For that purpose, 1,1,1-trichloroethane, stabilized in accordance with example 7 of German Published Application 29 26 000, having the composition:

|    |                      |         |
|----|----------------------|---------|
|    | 1,1,1-trichloroethane | 94.995% |
| a) | tert-butyl alcohol   | 2.5 %   |
| b) | trioxane             | 1.4 %   |
| c) | 1,2-butylene oxide   | 0.4 %   |
| d) | nitromethane         | 0.7 %   |
| e) | N-methyl morpholine  | 0.005%  |

is taken and subjected to the BAM test with fractional distillation.

It is found that 1,3,5-trioxane is concentrated in the 3rd distillation fraction, in accordance with its relatively high boiling point.

The trioxane content of this fraction is reduced as follows in the course of the BAM test:

| | |
|---|---|
| start of experiment | 100% trioxane |
| after 16 h refluxing | 15% trioxane |
| after 24 h refluxing: less than | 1% trioxane. |

During this time, particularly between 16 h and 24 h, HCl is continuously liberated. This is a disadvantage, in as much as it gives rise to intense corrosion under adverse conditions of use. Moreover, the volatility of the trioxane decomposition products represents an extreme load on the environment, which gives rise to unavoidable exposures, particularly during work, to open degreasing belts.

Similar results are obtained when BAM tests 2 and 3 are performed.

Under analogous experimental conditions, on the other hand, there is virtually no decomposition when the cyclic ether constituent 1,4-dioxane according to the invention is used: the 1,4-dioxane content is still above 90% of the initial value after being refluxed for 24 h and moreover, there is no identifiable liberation of HCl.

When BAM tests 2 and 3 performed, no decomposition whatsoever of the 1,4-dioxane can be detected.

In the comparative examples 1 to 5 and the examples according to the invention stated hereinafter, 1,1,1-trichloroethane was mixed with the constituents stated in each case and examined in accordance with the aforesaid test method:

Comparative Example 1:

| 1,1,1-trichloroethane with | % wt. |
|---|---|
| nitromethane | 1 |
| tert-butanol | 5 |
| butylene oxide | 0.5 |

Results of BAM test:
BAM 1: passed
BAM 2: passed
BAM 3: passed
BAM 1/fraction 1: passed
fraction 2: passed
fraction 3: after 6 h reaction.

3

Analysis of the 3rd distillation fraction showed depletion of tert-butanol to 2.9% wt. and of nitromethane to 0.8% wt.

Comparative Example 2:

| | 1,1,1-trichloroethane with | % wt. |
|---|---|---|
| | 1,4-dioxane | 1 |
| | tert-butanol | 5 |
| | butylene oxide | 0.5 |

Results of BAM test:
BAM 1: reaction after 10 h
BAM 2: passed
BAM 3: reaction after 14 h

Comparative Example 3:

| | 1,1,1-trichloroethane with | % wt. |
|---|---|---|
| | 1,4 dioxane | 5 |
| | nitromethane | 1 |
| | butylene oxide | 0.5 |

Results of BAM test:
BAM 1: passed
BAM 2: passed
BAM 3: passed
BAM 1/fraction 1: reaction after 4 h.

Analysis of the 1st distillation fraction showed depletion of dioxane to 2.5% wt. and augmentation of nitromethane to 1.3% wt.

Comparative Example 4:

| | 1,1,1-trichloroethane with | % wt. |
|---|---|---|
| | tert-butanol | 3.3 |
| | 1,4-dioxane | 1.7 |
| | nitromethane | 0.5 |
| | diisopropylamine | 0.03 |

Results of BAM test:
BAM 1: passed
BAM 2: passed
BAM 3: reaction after 10 h

Comparative Example 5:

| | 1,1,1-trichloroethane with | % wt. |
|---|---|---|
| | tert-butanol | 3.3 |
| | 1,4-dioxane | 1.7 |
| | nitromethane | 0.3 |
| | butylene oxide | 0.5 |

Results of BAM test:
BAM 1: passed
BAM 2: passed
BAM 3: reaction after 6 h

Example 1:

| | 1,1,1-trichloroethane with | % wt. |
|---|---|---|
| | tert-butanol | 3.7 |
| | 1,4-dioxane | 1.8 |
| | 1,2-butylene oxide | 0.5 |
| | nitromethane | 0.6 |
| | diisopropylamine | 0.02 |

Results of BAM test:
BAM 1: passed
BAM 2: passed
BAM 3: passed
BAM 1/fraction 1: passed
fraction 2: passed
fraction 3: passed

Example 2

| 1,1,1-trichloroethane with | % wt. |
|---|---|
| tert-butanol | 3.7 |
| 1,4-dioxane | 1.8 |
| 1,2-butylene oxide | 0.2 |
| cyclohexene oxide | 0.3 |
| nitromethane | 0.6 |
| triethylamine | 0.03 |

Results of BAM test:

BAM 1: passed
BAM 2: passed
BAM 3: passed
BAM 1/fraction 1: passed
fraction 2: passed
fraction 3: passed

**Claims**

1. Stabilized 1,1,1-trichloroethane characterized in that it comprises a combination of:
3 —5 % wt. tert-butanol,
1.5 —2.5 % wt. 1,4-dioxane,
0.3 —0.8 % wt. nitroalkane with 1 to 3 carbon atoms,
0.3 —0.8 % wt. butylene oxide and/or cyclohexene oxide and
0.01—0.05% wt. aliphatic and/or heterocyclic amine as stabilizers.
2. Stabilized 1,1,1-trichloroethane as claimed in claim 1, characterized in that it comprises nitromethane.
3. Stabilized 1,1,1-trichloroethane as claimed in claims 1 or 2, characterized in that it comprises diisopropylamine.
4. Stabilized 1,1,1-trichloroethane as claimed in any one of the claims 1 to 3, characterized in that it comprises 3.7% wt. tert-butanol, 1.8% wt. 1,4-dioxane, 0.5% wt. 1,2-butylene oxide, 0.6% wt. nitromethane and 0.02% wt. diisopropylamine.
5. Stabilized 1,1,1-trichloroethane as claimed in claims 1 or 2, characterized in that it contains 3.7% wt. tert-butanol, 1.8% wt. 1,4-dioxane, 0.2% wt. 1,2-butylene oxide, 0.3% wt. cyclohexene oxide, 0.6% wt. nitromethane and 0.03% wt. trimethylamine.
6. Synergistic stabilizer composition for application of stabilized 1,1,1-trichloroethane, characterized in that said composition comprises tert-butanol, 1,4-dioxane, a nitroalkane with 1 to 3 carbon atoms, butylene oxide and/or cyclohexene oxide and an aliphatic and/or heterocyclic amine.

**Patentansprüche**

1. Stabilisiertes 1,1,1-Trichlorethan, dadurch gekennzeichnet, daß es als Stabilisatoren eine Kombination aus:
3 bis 5 Gew.-% tert. Butanol,
1,5 bis 2,5 Gew.-% 1,4-Dioxan,
0,3 bis 0,8 Gew.-% Nitroalkan mit 1 bis 3 Kohlenstoffatomen,
0,3 bis 0,8 Gew.-% Butylenoxid und/oder Cyclohexanoxid und
0,01 bis 0,05 Gew.-% aliphatischem und/oder heterocyclischem Amin
enthält.
2. Stabilisiertes 1,1,1-Trichlorethan nach Anspruch 1, dadurch gekennzeichnet, daß es Nitromethan enthält.
3. Stabilisiertes 1,1,1-Trichlorethan nach Anspruch 1 oder 2, dadurch gekennzeichnet, daß es Diisopropylamin enthält.
4. Stabilisiertes 1,1,1-Trichlorethan nach einem der Ansrpüche 1 bis 3, dadurch gekennzeichnet, daß es 3,7 Gew.-% tert. Butanol, 1,8 Gew.-% 1,4-Dioxan, 0,5 Gew.-% Butylenoxid-1,2, 0,6 Gew.-% Nitromethan und 0,02 Gew.-% Diisopropylamin enthält.
5. Stabilisiertes 1,1,1-Trichlormethan nach Anspruch 1 oder 2, dadurch gekennzeichnet, daß es 3,7 Gew.-% tert.-Butanol, 1,8 Gew.-% 1,4-Dioxan, 0,2 Gew.-% Butylenoxid-1,2, 0,3 Gew.-% Cyclohexanoxid, 0,6 Gew.-% Nitromethan und 0,03 Gew.-% Triethylamin enthält.
6. Synergistische Stabilisatorzusammensetzung zur Anwendung von stabilisiertem 1,1,1-Trichlorethan, dadurch gekennzeichnet, daß die Zusammensetzung tert. Butanol, 1,4-Dioxan, ein Nitroalkan mit 1 bis 3 Kohlenstoffatomen, Butylenoxid und/oder Cyclohexanoxid und ein aliphatisches und/oder heterocyclisches Amin umfaßt.

**0 091 708**

**Revendications**

1. 1,1,1-Trichloroéthane stabilisé, caractérisé en ce qu'il comprend une combinaison de:
3—5% en poids de tert-butanol,
1,5—2,5% en poids de 1,4-dioxanne,
0,3—0,8% en poids de nitroalcane avec 1 à 3 atomes de carbone,
0,3—0,8% en poids d'oxyde de butylène et/ou d'oxyde de cyclohexéne et
0,01—0,05% en poids d'amine aliphatique et/ou hétérocyclique, à titre de stabilisants.

2. 1,1,1-trichloroéthane stabilisé selon la revendication 1, caractérisé en ce qu'il comprend du nitrométhane.

3. 1,1,1-trichloroéthane stabilisé selon la revendication 1 ou 2, caractérisé en ce qu'il comprend de la diisopropylamine.

4. 1,1,1-trichloroéthane stabilisé selon l'une quelconque des revendications 1 à 3, caractérisé en ce qu'il comprend 3,7% en poids de tert-butanol, 1,8% en poids de 1,4-dioxanne, 0,5% en poids d'oxyde de 1,2-butylène, 0,6% en poids de nitrométhane et 0,02% en poids de diisopropylamine.

5. 1,1,1-trichloroéthane stabilisé selon la revendication 1 ou 2, caractérisé en ce qu'il contient 3,7% en poids de tert-butanol, 1,8% en poids de 1,4-dioxanne, 0,2% en poids d'oxyde de 1,2-butylène, 0,3% en poids d'oxyde de cyclohexène, 0,6% en poids de nitrométhane et 0,03% en poids de triéthylamine.

6. Composition stabilisante synergique pour l'application du 1,1,1-trichloroéthane stabilisé, caractérisée en ce que ladite composition comprend du tert-butanol, du 1,4-dioxanne, un nitroalcane avec 1 à 3 atomes de carbone, de l'oxyde de butylène et/ou de l'oxyde de cyclohexène et une amine aliphatique et/ou hétérocyclique.

6